# EUROPÄISCHE PATENTSCHRIFT

(11) **EP 3 463 823 B1**
(45) Veröffentlichungstag und Bekanntmachung des Hinweises auf die Patenterteilung: **23.09.2020**
(21) Anmeldenummer: 17701094.9
(22) Anmeldetag: 17.01.2017
(51) Int. Cl.: B29C 67/04, B29C 67/00, B29C 43/00, A61L 27/00, A61F 2/30

(54) **GRANULATHERSTELLUNG MIT GERUNDETEN PARTIKELN FÜR DIE IMPLANTATFERTIGUNG ODER WERKZEUGFERTIGUNG**
GRANULATE PRODUCTION WITH ROUNDED PARTICLES FOR MANUFACTURING IMPLANTS OR TOOL MANUFACTURING
RÉALISATION DE GRANULÉS À PARTICULES ARRONDIES POUR LA FABRICATION D'IMPLANT OU LA FABRICATION D'OUTIL

(30) Priorität: 07.06.2016 DE 102016110501
(43) Veröffentlichungstag der Anmeldung: 10.04.2019
(73) Patentinhaber: Karl Leibinger Medizintechnik GmbH & Co. KG, 78570 Mühlheim (DE)
(72) Erfinder: AKSU, Adem, 78054 Villingen-Schwenningen (DE); REINAUER, Frank, 78576 Emmingen-Liptingen (DE); WOLFRAM, Tobias, 63303 Dreieich (DE)
(74) Vertreter: Winter, Brandl, Fürniss, Hübner, Röss, Kaiser, Polte - Partnerschaft mbB
(86) Internationale Anmeldenummer: PCT/EP2017/050894
(87) Internationale Veröffentlichungsnummer: WO 2017/211469

(56) Entgegenhaltungen:
- WO-A1-03/064141
- WO-A1-2009/014718
- US-B1- 6 641 617

## Beschreibung

Die Erfindung betrifft ein Verfahren zum Herstellen eines Kunststoff aufweisenden, vorzugsweise aus Kunststoff bestehenden, sowie zur chirurgischen Anwendung vorgesehenen Gegenstandes / Utensils, insbesondere eines Implantats oder eines chirurgischen Werkzeuges / Hilfsmittels. Auch betrifft die Erfindung ein Implantat, ein Hilfsmittel und ein Werkzeug.

Aus dem Stand der Technik ist es bereits prinzipiell bekannt, UHMWPE-Materialien ("Ultra High Molecular Weight Polyethylen"- / Ultrahochmolekulare Polyethylen-Materialien) für das Herstellen von Implantaten zu verwenden. So offenbart bspw. die US 6 641 617 B1 ein medizinisches Implantat zum Gebrauch in einem Körper, wobei dieses Implantat aus diesem UHMWPE-Material geformt ist.

WO 2009/014718 A1 offenbart ein Verfahren zum Herstellen eines porösen Implantats, umfassend: Bereitstellen eines Granulats aus einem Kunststoff (PE, PP, HDPE, UHMWPE); Verbinden des Granulats zu einem einstöckigen Grundkörper durch selektives Lasersintern.

Als nachteilig hat es sich jedoch bei den bereits bekannten Verfahren gezeigt, dass die Herstellung der medizinischen Gegenstände, die in der Chirurgie angewendet werden, bspw. von Implantaten, oft relativ aufwändig ist. Dies liegt insbesondere daran, dass das Ausgangs-Kunststoff-Material eine spezifische Form (Korngröße, Kornform, etc.) und gleichzeitig eine relativ hohe Viskosität aufzuweisen hat. Im Stand der Technik wird daher für die Herstellung von Implantaten aus Kunststoff meist überwiegend auf chemische Verfahrensschritte, durch Verwendung unterschiedlicher Lösungsmittel, zurückgegriffen.

Es ist die Aufgabe der vorliegenden Erfindung, die aus dem Stand der Technik bekannten Nachteile zu beheben und insbesondere ein Verfahren zur Herstellung eines medizinischen Gegenstandes aus Kunststoff zur Verfügung zu stellen, dessen Aufwand deutlich reduziert sein soll.

Dies wird erfindungsgemäß durch ein Verfahren gemäß des ersten Anspruchs gelöst, wobei zum Herstellen eines aus Kunststoff bestehenden sowie zur chirurgischen

Anwendung vorgesehenen Gegenstandes, insbesondere eines Implantats oder eines chirurgischen / medizinischen Hilfsmittels, wie eines (Operations-)Werkzeuges, zumindest folgende Schritte umgesetzt werden:
a) Bereitstellen (einer bestimmten Menge (Volumen oder Gewicht)) eines (rieselfähigen) Kunststoff-Pulvers;
b) Erhitzen und Verpressen des Kunststoff-Pulvers unter Ausbildung zumindest eines Zwischenstücks / zu zumindest einem Zwischenstück;
c) Mechanisches Zerkleinern des zumindest einen Zwischenstücks zu Granulat (von vorzugsweise vorbestimmter Korngröße und/oder Kornform); und
d) Verbinden des Granulats zu einem Grundkörper.

Auch wird die Aufgabe durch ein (vorzugsweise plattenförmiges) Implantat mit zumindest einem ein UHMWPE-Material aufweisenden, porösen Grundkörper gelöst.

Durch die genannten Verfahrensschritte wird der Gegenstand weitgehend ohne chemische Reaktionsschritte, etwa durch Lösungen, und überwiegend oder vollständig durch mechanische bzw. physikalische Bearbeitungsschritte bereitgestellt. Durch das Verpressen des Kunststoff-Pulvers in Zwischenstücke sowie das anschließende mechanische Zerkleinern können definierte sowie einheitliche Partikel als Granulat verwendet werden, sodass ein möglichst reproduzierbares Herstellverfahren umgesetzt wird. Insbesondere kann dadurch die beabsichtigte Porosität des herzustellenden Gegenstandes, vorzugsweise des Implantates gezielt eingestellt werden. Durch den weitest gehenden Verzicht auf üblicherweise biologisch aufwändig abzubauenden Reaktionsmitteln wird die Umweltbelastung wesentlich verringert.

Weitere vorteilhafte Ausführungsformen sind in den Unteransprüchen beansprucht und nachfolgend näher erläutert.

Der zur chirurgischen Anwendung vorgesehene Gegenstand ist vorteilhafterweise ein Implantat, das weiter bevorzugt zur Osteosynthese oder Frakturversorgung und/oder zur Ausbildung eines (patientenspezifischen) Individualimplantats dient. In weiteren Ausführungen ist es auch zweckmäßig, wenn der zur chirurgischen Anwendung vorgesehene Gegenstand als ein Operations-Hilfsmittel, etwa ein (Operations-) Werkzeug/Tool, für die medizinische Anwendung während einer Operation ausgebildet ist.

Das Kunststoff-Pulver besteht vorzugsweise aus einem einzigen Kunststoff-Material. Das Herstellungsverfahren ist dann besonders einfach reproduzierbar.

Dabei ist es besonders vorteilhaft, wenn das Kunststoff-Pulver aus einem ThermoplastMaterial, bevorzugt einem Polyethylen (PE), besonders bevorzugt einem Ultrahochmolekularen Polyethylen (UHMWPE), weiter bevorzugt einem hochdichten Polyethylen (HDPE), noch weiter bevorzugt aus einem Polypropylen (PP), etwa einem Polypropylen-Fumarat (PPF), oder noch weiter bevorzugt aus einem Polyaryletherketon (PAEK), insbesondere einem Polyetheretherketon (PEEK), besteht. Dadurch wird der Gegenstand, vorzugsweise ein Implantat, auf die jeweiligen Einsatzgebiete optimal materiell abgestimmt.

Auch ist es vorteilhaft, wenn das Kunststoff-Pulver aus einem Duroplast-Material, besonders bevorzugt aus einem biologisch abbaubaren / bioverträglichen Duroplast-Material, bevorzugt einem (duroplastischen) Polyurethan (PUR), weiter bevorzugt einem Polyacrylat oder einem Epoxidharz, besteht. Auch dann können insbesondere leistungsfähige Implantate als Gegenstand erzeugt werden.

Zudem ist es insbesondere für die Herstellung eines Hilfsmittels, wie eines Werkzeuges, für den Gegenstand vorteilhaft, wenn das Kunststoff-Pulver aus einem Elastomer-Material, bevorzugt einem (elastomeren) Polyurethan (PUR), weiter bevorzugt einem Silikon-Material, besonders bevorzugt einem Polysulfid, besteht.

Weiter vorteilhaft ist es jedoch auch, wenn das Kunststoff-Pulver aus verschiedenen Kunststoff-Materialien, d.h. aus verschiedenen Thermoplast-, Duroplast- und/oder Elastomer-Materialien, besteht. Dabei ist es besonders bevorzugt, wenn das Kunststoffpulver aus einem UHMWPE mit HDPE- und/oder PE- Beimischungen besteht. Eine solche Pulvermischung ist besonders für die Herstellung von Gegenständen als Implantate geeignet. Eine weitere Beimischung von PP, PPF, PAEK, wie PEEK, (elastomeren und/oder duroplastischem) PUR, Polyacrylat und / oder Epoxidharz zu diesem UHMWPE-HDPE-PE-Gemisch macht den Einsatz des Gegenstandes weiter flexibel.

Weiter vorteilhaft ist es, wenn der Grundkörper eine poröse Struktur aufweist. Eine solche poröse Struktur ist besonders günstig für die Akzeptanz / das Einwachsen eines als medizinisches Implantat ausgebildeten Gegenstandes im Körper des jeweiligen Säugers.

In diesem Zusammenhang ist es zudem zweckmäßig, wenn die poröse Struktur eine offen- oder geschlossenzellige / -porige Struktur ist, d.h. eine interkonnektierende und / oder nicht interkonnektierende Form aufweist. Einerseits kann durch eine offenporige Struktur das Einwachsen des Gegenstands im Körper des Säugers weiter gefördert werden, durch eine geschlossenporige Struktur hingegen die Festigkeit weiter erhöht werden.

In diesem Zusammenhang ist es besonders vorteilhaft, wenn der Grundkörper eine derartige Porosität aufweist, dass die Porengrößen zwischen 10 µm bis 450 µm liegen, weiter bevorzugt kleiner als 300 µm sind / zwischen 10 µm und 300 µm liegen, besonders bevorzugt zwischen 200 µm und 300 µm liegen. Auch ist es vorteilhaft, wenn der Grundkörper eine derartige Porosität aufweist, dass die Porengrößen zwischen 500 µm und 850 µm liegen. Dadurch ist der Gegenstand für den medizinischen Einsatz weiter optimiert.

Weiter ist es vorteilhaft, wenn der Grundkörper eine Porosität zwischen 30 und 45 %, weiter bevorzugt zwischen 50 und 60 %, besonders bevorzugt von größer als 80 % aufweist. Dadurch ist der Grundkörper besonders leistungsfähig.

In diesem Zusammenhang ist es weiterhin auch vorteilhaft, wenn dem Kunststoff-Pulver (vor Durchführen des Schrittes a) oder d)) mindestens ein weiterer Zusatzstoff, bevorzugt ein proosteosynthetischer Zusatzstoff hinzugefügt wird. Dieser Zusatzstoff ist vorzugsweise ein Hydroxylapatit (HAP), ein Calciumkarbonat (CaCO₃), ein Magnesium (Mg), ein Eisen (Fe), ein Strontium (Sr), ein alpha- oder beta-Tricalciumphosphat (alpha / beta TCP), Bioglass®-Partikel / Partikel aus bioaktivem Glas, ein Polyestermaterial, wie PDLLA, PLGA, PLA, PGA, Chitosan-Fasern oder ein Chitosan-Partikel. Dadurch lassen sich besonders bioverträgliche sowie stabile Gegenstände erzeugen.

Ist der Grundkörper desweiteren hydrophylisiert, ist der Gegenstand als Implantat auch weiter für seinen Einsatz in einem menschlichen Körper optimiert.

Zudem ist es von Vorteil, wenn die Verfahrensschritte a) bis d) zeitlich aufeinander folgend stattfinden. Dadurch ist das Verfahren besonders effizient ausgebildet.

Weist das Kunststoff-Pulver eine Korngröße zwischen ca. 20 µm oder ca. 50 µm und ca. 900 µm, vorzugsweise zwischen ca. 300 µm und ca. 600 µm, weiter vorzugsweise ca. 500 µm ± 100 µm auf ist die Herstellung der Zwischenstücke einfach umsetzbar.

Besonders vorteilhaft ist es, wenn das zumindest eine Zwischenstück plattenförmig ausgebildet ist, (zumindest abschnittsweise oder vollständig) eine poröse Materialstruktur aufweist und/oder (zumindest abschnittsweise oder vollständig) aus einem Vollmaterial gebildet ist. Dadurch ist das Zwischenstück in seiner Ausgestaltung günstig für die anschließende Zerkleinerung vorbereitet.

Zweckmäßig ist es desweiteren, wenn Schritt c) einen ersten Teilschritt c1), in dem das zumindest eine Zwischenstück, vorzugsweise spanend, schneidend und/oder stanzend, in Einzelstücke vorzerkleinert wird, und/oder einen zweiten Teilschritt c2), in dem die Einzelstücke in Granulat / Pellets, vorzugsweise mittels Zermahlen, (weiter) zerkleinert werden, aufweist. Dadurch kann das Granulat in Form und Größe besonders präzise hergestellt werden.

Weiterhin ist es vorteilhaft, wenn in dem zweiten Teilschritt c2) das Zermahlen mittels einer Rotormühle durchgeführt wird, wobei ein Rotor und/oder ein Sieb der Mühle vorzugsweise als ein Conidur®-Blech oder Blech mit mehreren Löchern, etwa Rundlöchern, ausgebildet ist. Dadurch kann eine endgültige Form des Granulats besonders geschickt umgesetzt werden. Es ist aber empfehlenswert dabei flüssigen Stickstoff zu verwenden, weil dann eine Verklebung im Sieb oder im Rotor vermieden wird. Besonders bewährt haben sich Rotorformen nach Art einer Schlagnase und eines Turborotors in Kombination mit einem Rundlochsieb und/oder einem Conidur-Sieb, wobei diese gegenüber den Schlitzlochblechen eine mehr dreieckförmige bis halbelliptische Öffnung haben.

In diesem Zusammenhang ist es besonders vorteilhaft, wenn die Form der Partikel des Granulats (vorzugweise einheitlich) rund, oval, dreieckförmig und/oder rechteckförmig ausgestaltet ist. Insbesondere ist es vorteilhaft, wenn die Partikel jeweils eine abgerundete Oberfläche, d.h. abgerundete Kanten aufweisen. Somit ist der Gegenstand besonders dauerfest hergestellt.

Zweckmäßig ist es in Bezug auf den zweiten Teilschritt c2) auch, wenn die Partikel des Granulats nach Durchführen des zweiten Teilschrittes c2) eine Korngröße zwischen 20 µm und 2000 µm aufweisen. Derartige Korngrößen sind für den Einsatz des Gegenstandes als medizinisches Implantat besonders geeignet.

Umfasst Schritt d) ein Sintern des Granulats, vorzugsweise ein poröses Sintern und/oder ein selektives Lasersintern, d.h. wird Schritt d) als ein Sinter-Vorgang durchgeführt, ist der Gegenstand gut automatisiert herstellbar.

In diesem Fall wird der Sintervorgang an sich vorzugsweise in einer Stickstoff- und / oder Argon-Atmosphäre oder in einem Vakuum / unter Vakuum durchgeführt. Auch kann desweiteren eine Membran beim Sintern eingesetzt werden, wodurch das Erzeugen der Atmosphäre / des Vakuums besonders effektiv umgesetzt ist und eine gewisse Elastizität vorhanden ist. Dadurch wird das Herstellverfahren noch leistungsfähiger.

Nach dem Sintern oder während des Sinterns kann auch ein zusätzliches Verpressen oder eine zusätzliche Auslaugung (leaching) unterschiedlicher Materialkomponenten / des Kunststoff-Materials des Grundkörpers durchgeführt werden, wodurch sich die Porosität des Gegenstandes lokal oder gesamtheitlich verändert. In diesem Zusammenhang ist es auch besonders vorteilhaft, wenn während des Sinterns ein biodegradierbarer Werkstoff, wie ein HAP, CaCO₃, alpha / beta / x-TCP, etc., eingefüllt wird und entsprechend mit dem Granulat aus dem Kunststoff-Material vernetzt wird. Dadurch lassen sich besonders geschickt die mechanischen Eigenschaften des Gegenstandes einstellen. Die Partikel können, wie bereits erwähnt, unterschiedliche Formen aufweisen, wobei sie vorzugsweise eine abgerundete Form aufweisen, um somit einen optimalen Energieeintrag während des Sinterns zur Verfügung zu stellen.

Weiterhin ist es vorteilhaft, wenn an dem Grundkörper, vorzugsweise zeitlich nach dem Schritt d) folgend, in einem Schritt e) eine Sterilisationsbestrahlung des Grundkörpers derart durchgeführt wird, dass sich das Kunststoff-Material (zusätzlich) vernetzt. Dadurch wird die Stabilität des Gegenstands weiter verbessert.

In diesem Zusammenhang ist es besonders vorteilhaft, wenn eine Gamma-Sterilisationsbestrahlung mit vorzugsweise 10 bis 45 kGy, weiter bevorzugt mit rund 25 kGy (entsprechend 2,5 Milliarden rd), eine Sterilisationsbestrahlung /-bedampfung mit ETO-Gas, eine E-Beam-Sterilisation, oder eine Plasmasterilisation durchgeführt wird. Dadurch lässt sich auch die Bestrahlung besonders gut automatisierbar umsetzen.

Wird der Grundkörper desweiteren, vorzugsweise zeitlich nach den Schritten d) und e) oder zwischen den Schritten d) und e), in einem Schritt f) gereinigt, wird die Qualität des Gegenstands / des Grundkörpers weiter verbessert.

Es ist von Vorteil, wenn der Grundkörper gereinigt wird, beispielsweise mittels Schneebestrahlen, etwa unter Einsatz von gefrorenem CO₂.

Dabei ist es besonders zweckmäßig, wenn das Reinigen des Grundkörpers eine Ultraschallbadreinigung und / oder einen Oberflächenbehandlung, etwa eine Oberflächenbehandlung, wie ein Schneestrahlen, mittels CO2 basierten Technologien, oder eine thermische Oberflächennachbehandlung aufweist. Die Ultraschallbadreinigung wird weiter bevorzugt mittels Ethanol oder Isopropanol durchgeführt. Somit wird ein besonders hoher Reinigungsgrad erreicht.

Auch ist es von Vorteil, wenn, vorzugsweise zeitlich nach den Schritten d), e) und/oder f), in einem weiteren Schritt (vorzugsweise einem Schritt g)) eine (vorzugsweise thermische) Oberflächenbehandlung des Grundkörpers durchgeführt wird. Dadurch lassen sich alle Partikel des Grundkörpers besonders dauerhaft befestigen.

Weist die Oberflächenbehandlung eine Plasma- / Niederdruckplasma-Oberflächenbehandlung (in Form einer Wärmenachbehandlung) auf, wird ein besseres Einwachsverhalten erzielt. Insbesondere wird dabei eine Festigkeitssteigerung von UHMWPE-, HDPE-, und PP-Implantaten sowie eine Fixierung der Restpartikel auf der Oberfläche erreicht.

Wenn die Oberflächenbehandlung, zusätzlich oder alternativ zur Plasma- / Niederdruckplasma-Oberflächenbehandlung, eine Heißlufttemperaturbehandlung auf, vorzugsweise durch einen Heißluftföhn, besteht zudem die Möglichkeit der nachträglichen intraoperativen Formgestaltung durch eine Wärmebehandlung, sodass wiederum eine Festigkeitssteigerung von UHMWPE-, HDPE-, und PP-Implantaten durch eine Wärmenachbehandlung und Fixierung der Restpartikel auf der Oberfläche erzielt wird.

Weist die Oberflächenbehandlung, vorzugsweise zusätzlich zu der Heißluft-/ Heißlufttemperaturbehandlung, ein Explosionsentgraten auf einer speziellen Kunststoffanlage oder ähnlichem auf, besteht wiederum die Möglichkeit der nachträglichen intraoperativen Formgestaltung durch Wärmebehandlung, sodass eine Festigkeitssteigerung von UHMWPE-, HDPE-, und PP-Implantaten durch eine Wärmenachbehandlung, wie Heißluftföhn, und eine Fixierung der Restpartikel auf der Oberfläche (somit interkonnektierende Festigkeitssteigerung) erzielt wird.

Falls ferner die Oberflächenbehandlung zusätzlich oder alternativ eine Behandlung der Oberfläche des Grundkörpers mit überkritischem CO₂ aufweist, wird eine weitere Möglichkeit der nachträglichen intraoperativen Formgestaltung durch Wärmebehandlung geschaffen und die Festigkeitssteigerung von UHMWPE-, HDPE-, und PP-Implantaten durch eine Wärmenachbehandlung sowie eine Fixierung der Restpartikel auf der Oberfläche, d.h. eine interkonnektierende Festigkeitssteigerung, fortgebildet.

Weist die Oberflächenbehandlung zusätzlich oder alternativ eine Behandlung der Oberfläche des Grundkörpers mit Infrarotlicht durch Infrarotstrahler auf, wird eine weitere Möglichkeit der nachträglichen intraoperativen Formgestaltung durch Wärmebehandlung geschaffen und die Festigkeitssteigerung von UHMWPE-, HDPE-, und PP-Implantaten durch eine Wärmenachbehandlung sowie eine Fixierung der Restpartikel auf der Oberfläche, d.h. eine interkonnektierende Festigkeitssteigerung, fortgebildet.

Desweiteren ist es von Vorteil, wenn die Oberflächenbehandlung zusätzlich oder alternativ eine Beflammung des Grundkörpers aufweist, wird eine weitere Möglichkeit der nachträglichen intraoperativen Formgestaltung durch Wärmebehandlung geschaffen und die Festigkeitssteigerung von UHMWPE-, HDPE-, und PP-Implantaten durch eine Wärmenachbehandlung sowie eine Fixierung der Restpartikel auf der Oberfläche, d.h. eine interkonnektierende Festigkeitssteigerung, fortgebildet.

Beinhaltet die Oberflächenbehandlung zusätzlich oder alternativ eine Wärmebehandlung in einem Wärmeofen auf, wird eine weitere Möglichkeit der nachträglichen intraoperativen Formgestaltung durch Wärmebehandlung geschaffen und die Festigkeitssteigerung von UHMWPE-, HDPE-, und PP-Implantaten durch eine Wärmenachbehandlung sowie eine Fixierung der Restpartikel auf der Oberfläche, d.h. eine interkonnektierende Festigkeitssteigerung, fortgebildet.

In diesem Zusammenhang ist es besonders vorteilhaft, wenn die Oberflächenbehandlung, insbesondere die Heißluftbehandlung, die Beflammung, und/oder die Plasma- Oberflächenbehandlung, mittels eines Roboterarmes durchgeführt wird. Dadurch wird eine weitere Möglichkeit der nachträglichen intraoperativen Formgestaltung durch Wärmebehandlung geschaffen und die Festigkeitssteigerung von UHMWPE-, HDPE-, und PP-Implantaten durch eine Wärmenachbehandlung sowie eine Fixierung der Restpartikel auf der Oberfläche, d.h. eine interkonnektierende Festigkeitssteigerung, fortgebildet.

Prinzipiell sei auch darauf hingewiesen, dass ein Grundkörper nach Durchführen der Verfahrensschritte aus dem unabhängigen Anspruch 1, nämlich den Schritten a) bis d), bereits einen vollständigen Gegenstand, wie das Implantat, umsetzt. Die in den abhängigen Ansprüchen zusätzlich durchgeführten Verfahrensschritte e) bis g) bilden den Grundkörper weiter und tragen somit bei, dass der letztendlich erhaltene Gegenstand noch effizienter für den Einsatz in einem Körper eines Säugers verbessert wird. Die Verfahrensschritte e) bis g) können gemeinsam oder unabhängig voneinander zusätzlich zu den Schritten a) bis d) durchgeführt werden.

Bei Ausbildung des Gegenstandes als Implantat wird der Grundkörper entweder beim Einsatz während einer Operation, auf übliche Weise, in seiner Form an die patientenspezifische Geometrie der Knochen und Knorpel angepasst, kann jedoch auch bereits beim Sintern (unmittelbar nach Schritt d)) die letztendliche patientenspezifische Form aufweisen. Diese Form wird im letzteren Fall dann bspw. mittels eines Scan-Verfahrens des betroffenen Knochenteils des Patienten erfasst und im Sinterschritt ausgebildet.

Die Erfindung betrifft auch ein Implantat oder ein Hilfsmittel mit zumindest einem ein UHMWPE-Material aufweisenden Grundkörper. Als Hilfsmittel qualifiziert sich eine Schablone / ein Template oder ein Werkzeug. Bei dieser Variante ist der Grundkörper nicht-porös / verschlossen, während er in der Variante als Implantat porös ausgebildet ist.

Ein bevorzugtes erfindungsgemäßes Verfahren zur Herstellung eines aus Kunststoff / Kunststoff-Material bestehenden sowie zur chirurgischen Anwendung vorgesehenen Gegenstandes wird nachfolgend anhand einer Figur in einem Ausführungsbeispiel näher beschrieben.

Es zeigen:
- Fig. 1: eine schematische Darstellung des Herstellverfahrens nach einem erfindungsgemäßen Ausführungsbeispiel,
- Fig. 2: eine mikroskopische detaillierte Schnittdarstellung eines Querschnittes durch einen fertigen Grundkörper eines ein Implantat ausbildenden Gegenstandes, wie er nach dem Herstellverfahren gemäß Fig. 1 hergestellt ist, wobei insbesondere die Form des verwendeten Granulats als Kugeln zu erkennen ist,
- Fig. 3: eine mikroskopisch detaillierte Schnittdarstellung eines Querschnittes durch einen fertigen Grundkörper eines ein Implantat ausbildenden Gegenstandes, wie er nach einem Herstellverfahren gemäß eines zweiten Ausführungsbeispiels hergestellt ist, wobei sich dieses Herstellverfahren durch Verwendung polygonal ausgeformter Partikel des Granulats von dem Herstellverfahren nach Fign. 1 und 2 unterscheidet, und
- Fig. 4: eine perspektivische Ansicht eines menschlichen Schädels zur Veranschaulichung der möglichen Anbringungsbereiche des hergestellten Gegenstandes / Implantats.

Die Figuren sind lediglich schematischer Natur und dienen ausschließlich dem Verständnis der Erfindung. Die gleichen Elemente sind mit denselben Bezugszeichen versehen.

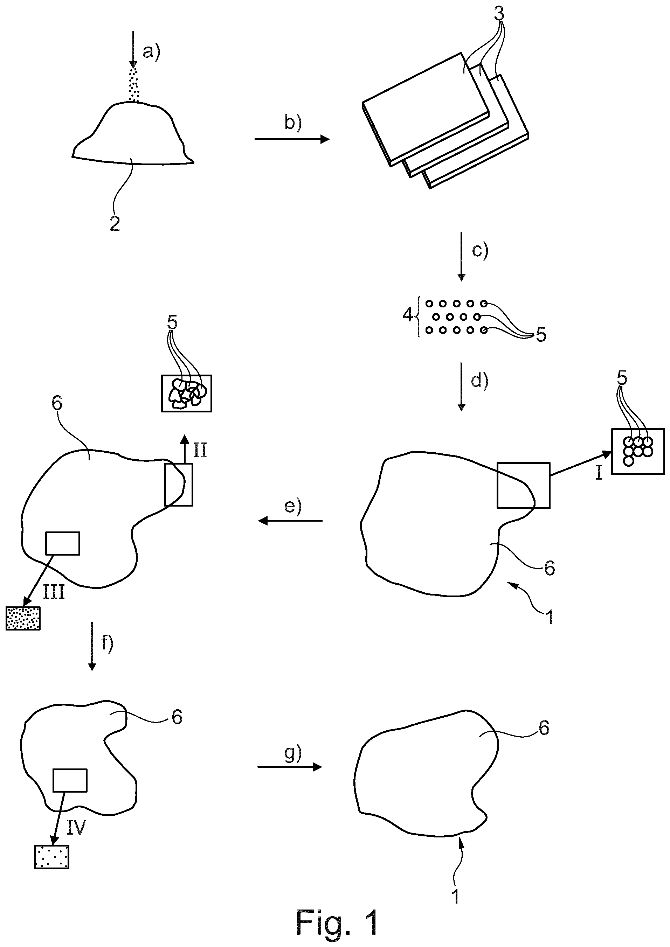

In Fig. 1 ist ein bevorzugtes erfindungsgemäßes Herstellverfahren gemäß eines ersten Ausführungsbeispiels gut zu erkennen. Zum Herstellen eines letztendlich fertig ausgebildeten Grundkörpers 6, der einen zur chirurgischen Anwendung vorgesehenen Gegenstand 1, nämlich ein medizinisches Implantat, ausbildet, werden in diesem Verfahren die Verfahrensschritte a) bis g), gekennzeichnet durch Pfeile, zeitlich nacheinander durchgeführt. Zum Herstellen des Grundkörpers 6 bedarf es zunächst das Durchführen der Verfahrensschritte a) bis d). Aufgrund der Ausbildung des Gegenstandes 1 als Implantat in den beiden nachfolgend beschriebenen Ausführungsbeispielen, ist nachfolgend das Implantat als der Gegenstand mit dem Bezugszeichen 1 versehen. Alternativ zu der Herstellung des Implantats 1 werden in weiteren Ausführungen auch andere Gegenstände, insbesondere Hilfsmittel für eine Operation, wie Operationswerkzeuge, durch dieses Herstellverfahren hergestellt.

Wie in Fig. 1 ersichtlich ist, wird zunächst ein Kunststoff-Pulver 2 in Form eines UHMWPE-Pulvers 2 bereitgestellt (Pfeil a)), wobei dieses Kunststoff-Pulver 2 eine Korngröße / durchschnittliche Korngröße von weniger als 300 µm aufweist.

Das rieselfähige Kunststoff-Pulver 2 wird daran im Anschluss, durch den Pfeil b) gekennzeichnet, mittels eines sinterähnlichen Verfahrens verpresst. Dabei entstehen einstückige / in sich zusammenhängende Zwischenstücke 3. Insbesondere werden die Zwischenstücke 3 durch ein Verpressen mit gleichzeitigem Erhitzen des Kunststoff-Pulvers 2 erreicht, wobei die Zwischenstücke 3 letztendlich rechteckförmige Platten ausbilden. Die Temperatur der Zwischenstücke 3 bei diesem Sintern / Urformen der Zwischenstücke 3 ist stets unterhalb der Zersetzungstemperatur des verwendeten Kunststoff-Pulvers 2 (bei mehreren Kunststoffmaterialien unterhalb der Zersetzungstemperatur der am niedrig schmelzenden Materialkomponente des verwendeten Kunststoff-Pulvers 2). Bevorzugt ist zur Herstellung des jeweiligen Zwischenstückes 3 eine Negativformen vorhanden, in die das Kunststoff-Pulver 2 zunächst eingefüllt wird und im Anschluss daran aufgeheizt sowie, mit einer Presskraft beaufschlagt, zusammengepresst wird, sodass sich ein in sich festes Gebilde in Form der Zwischenstücke 3 bildet.

Im Anschluss an das Fertigen der Zwischenstücke 3 wird gemäß Pfeil c) jedes Zwischenstück 3 wieder definiert zerkleinert. Die Zwischenstücke 3 werden in mehrere Partikel 5 unter Ausbildung eines Granulats 4 zerkleinert. Die Partikel 5 weisen eine im Wesentlichen einheitliche Form auf, die durch die konkrete Ausführung der mechanischen Zerkleinerung ausgeführt wird. In diesem Ausführungsbeispiel werden runde Partikel 5, in Form von kugelartigen oder im Querschnitt ovalen Partikeln 5, erzeugt.

Der Verfahrensschritt c) ist in zwei Teilschritte unterteilt, die hier der Übersichtlichkeit halber nicht weiter dargestellt sind. In einem ersten Teilschritt (als erster Teilschritt c1) bezeichnet) wird das zumindest eine Zwischenstück 3 schneidend vorzerkleinert, sodass aus einem Zwischenstück 3 wiederum eine Vielzahl von scharfkantigen Einzelstücken erzeugt wird. Alternativ, in weiteren Ausführungsbeispielen ist es auch angedacht, diese Einzelstücke statt durch das Schneiden oder zusätzlich zu dem Schneiden spanend, etwa fräsend oder drehend, und/oder stanzend herzustellen.

Im Anschluss an den ersten Teilschritt c1) werden die mehreren Einzelstücke in einem zweiten Teilschritt (als zweiter Teilschritt c2) bezeichnet) mechanisch weiter zerkleinert, nämlich vermahlen. Die Einzelstücke werden dabei derart lange vermahlen, bis sich das einheitliche, d.h. insbesondere in Größe und Form einheitliche, Granulat 4 aus einer Vielzahl von Partikeln 5 bildet. Das Mahlverfahren wird vorzugsweise mittels einer Rotormühle umgesetzt, wobei sich ein Rotor relativ zu einem gehäusefesten / feststehenden Bereich, nämlich einem Sieb, bewegt und die dazwischen angeordneten Einzelstücke aufgrund der mechanischen Scherkräfte zerkleinert werden. Rotor und Sieb weisen dann mehrere Löcher auf, die bereits die Umfangsgeometrie des fertigen Granulats 4 vorgeben. Da hier runde Partikel 5 ausgeformt werden, haben die Löcher / Durchgangslöcher ebenfalls eine runde Form. Durch hindurchdrücken der entsprechenden Einzelstücke durch die Löcher werden die Partikel 5 in ihre runde Form gebracht.

Gemäß Pfeil d) folgt im Anschluss das Verbinden des in seiner Form eingestellten Granulats 4 zu dem einteiligen Grundkörper 6. In diesem Ausführungsbeispiel dient zum Verbinden ein Sintervorgang, nämlich ein selektiver Laser-Sintervorgang. Alternativ hierzu ist es jedoch auch möglich, andere Sintertechniken, bspw. ein poröses Sintern oder gar andere Verbindungstechniken, etwa stoffschlüssige Verbindungstechniken, wie Schweißen, einzusetzen.

Nach Schritt d) besteht der Grundkörper 6 aus einem zusammenhängenden, stabilen Kunststoff-Material in Form des UHMWPE, das zuvor in Pulverform vorhanden war. Wie in der Teildarstellung der schematischen Ansicht nach Fig. 1 zwischen den Pfeilen d) und e) zu erkennen, wird ein beliebig ausgebildetes, im Wesentlichen plattenförmiges, Implantat 1 in Form dieses Grundkörpers 6 bereits vorausgeformt. In diesem Grundkörper 6 sind die einzelnen, zuvor rieselfähigen, Granulatpartikel 5 stoffschlüssig fest miteinander verbunden (Detaildarstellung "I"). Der Grundkörper 6 weist in diesem Verfahren bereits nach Durchführen des Schrittes d) im Wesentlichen die fertige Form des herzustellenden Implantates 1 auf. Das Implantat 1 ist hierbei auf typische Weise als ein Implantat 1 zur Osteosynthese bzw. Frakturversorgung ausgebildet, bspw. als ein Granialimplantat. Das Sintern wird derart ausgeführt, dass das Implantat / der Grundkörper 6 eine poröse, vorzugsweise eine offenporige Struktur aufweist. Alternativ sind auch geschlossenporige Strukturen umsetzbar.

Zusätzlich zu den Schritten a) bis d), die bereits zu einem fertigen Ausbilden des Implantats 1 / des Grundkörpers 6 dienen, sind in dem Ausführungsbeispiel nach Fig. 1 noch die Schritte e) bis g) umgesetzt. Mit dem Schritt e) wird der Grundkörper 6 anschließend an den Schritt d) noch einem Bestrahlungsvorgang, nämlich einer Sterilisationsbestrahlung ausgesetzt. Diese Sterilisationsbestrahlung dient einer zusätzlichen Vernetzung des UHMWPE-Materials, was in der auf den Pfeil e) folgenden Teildarstellung der Fig. 1 mittels eines Detailausschnittes "II" zu erkennen ist. Hierbei schmiegen sich die einzelnen Partikel 5 noch näher aneinander an bzw. vergrößern ihre Kontaktflächen zwischeneinander.

Nach der Sterilisationsbestrahlung gemäß Verfahrensschritt f) wird der Grundkörper 6 gereinigt, was zwischen den Teildarstellungen vor und nach dem Pfeil f) durch die Detaildarstellungen "III" und "IV" der Oberfläche erkennbar ist.

Nach dem Reinigen der Oberfläche wird mit Schritt g) eine thermische Oberflächennachbehandlung des Grundkörpers 6 durchgeführt. Dadurch ergibt sich letztendlich, dass nach dem Schritt g) fertige Implantat 1 gemäß dem bevorzugten Ausführungsbeispiel.

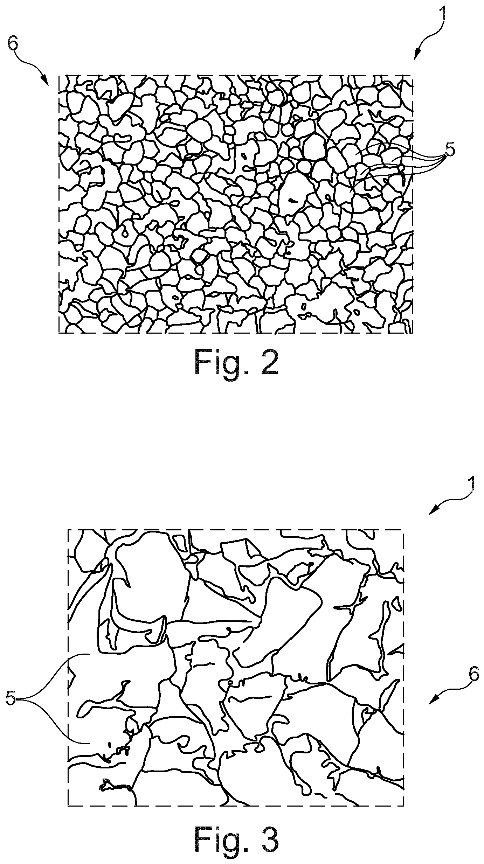

In Fig. 2 ist eine mikroskopische Detaildarstellung eines Querschnittes durch das fertige Implantat 1 aus Fig. 1 nochmals detailliert dargestellt. Hierin ist insbesondere die runde / ovale Querschnittsform der einzelnen Partikel 5 erkennbar.

In Verbindung mit Fig. 3 ist es jedoch auch möglich, andere Formen als diese runde Form prinzipiell vorzusehen. In Fig. 3, die einen Querschnitt eines anderen Implantats 1 darstellt, sind die Partikel 5 polygonal / mehreckig ausgeformt. Ein Implantat 1 mit einer solchen mehreckigen Ausgestaltung seiner Partikel 5 wäre mit einem ähnlichen Verfahren wie es in Fig. 1 dargestellt ist, ausführbar, wobei lediglich der Mahlvorgang gemäß Schritt c2) angepasst werden müsste. Anstatt runder Löcher im Rotor und im Sieb wären eckige Durchgangsöffnungen vorzusehen. Auch können diese in ihrer Größe variieren, sodass schließlich die Partikel 5 gemäß Fig. 3 etwas größer ausgestaltet sind als die in Fig. 2.

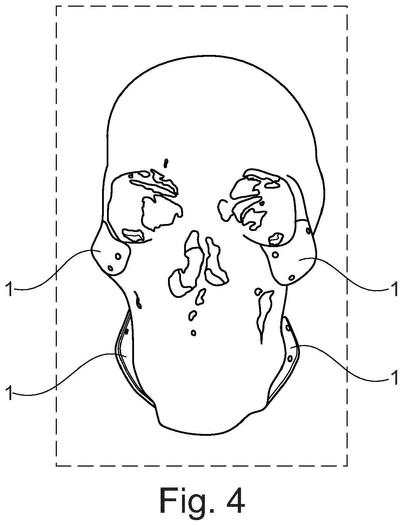

Das fertige Implantat 1 kann bspw. am Schädelknochen oder im Kieferbereich, wie in Fig. 4 erkennbar, oder ähnlichen Bereichen des Säugers eingesetzt werden. Auch kann das Implantat 1 / der Grundkörper 6 gemäß spezifischen Geometriedaten eines Patienten angefertigt werden. Hierfür ist es möglich, die entsprechende Sinterform bereits patientenspezifisch als Negativform auszubilden und somit nach Schritt d) bzw. nach Schritt g) bereits die fertige Form des Implantats 1 herzustellen. Alternativ hierzu ist es auch möglich, den fertigen Grundkörper 6 direkt bei der Operation noch geometrisch durch Biegen oder Schneiden in seiner Größe anzupassen.

In weiteren Ausführungen ist es auch möglich den Grundkörper 6 aus anderen Materialien als dem gewählten UHMWPE, etwa einem PE, einem PP oder einem HDPE herzustellen. Auch andere thermoplastische Materialien, duroplastische und/oder elastomere Kunststoffe sind zur Herstellung prinzipiell geeignet. Auch können Materialmischungen zur Herstellung gewählt werden, bspw. Mischungen aus UHMWPE, PP, PE und/oder HDPE.

### Bezugszeichenliste

- 1: Gegenstand / Implantat
- 2: Kunststoff-Pulver
- 3: Zwischenstück
- 4: Granulat
- 5: Partikel
- 6: Grundkörper

## Patentansprüche

1. Verfahren zum Herstellen eines Kunststoff aufweisenden sowie zur chirurgischen Anwendung vorgesehenen Gegenstandes (1) mit zumindest folgenden Schritten:
a) Bereitstellen eines Kunststoff-Pulvers (2);
b) Erhitzen und Verpressen des Kunststoff-Pulvers (2) unter Ausbildung zumindest eines Zwischenstücks (3);
c) Mechanisches Zerkleinern des zumindest einen Zwischenstücks (3) zu Granulat (4); und
d) Verbinden des Granulats (4) zu einem einstückigen Grundkörper (6).

2. Verfahren nach Anspruch 1, **dadurch gekennzeichnet, dass** der Grundkörper (6) eine poröse Struktur aufweist.

3. Verfahren nach Anspruch 2, **dadurch gekennzeichnet, dass** die poröse Struktur eine offen- oder geschlossenzellige Struktur ist.

4. Verfahren nach einem der Ansprüche 1 bis 3, **dadurch gekennzeichnet, dass** das Kunststoff-Pulver eine Korngröße zwischen 20 µm und 900 µm aufweist.

5. Verfahren nach einem der Ansprüche 1 bis 4, **dadurch gekennzeichnet, dass** das zumindest eine Zwischenstück (3) plattenförmig ausgebildet ist, eine poröse Materialstruktur aufweist und/oder aus Vollmaterial gebildet ist.

6. Verfahren nach einem der Ansprüche 1 bis 5, **dadurch gekennzeichnet, dass** der Schritt c) einen ersten Teilschritt c1), in dem das zumindest eine Zwischenstück (3) in Einzelstücke vorzerkleinert wird, und einen zweiten Teilschritt c2), in dem die Einzelstücke in Granulat (4) weiter zerkleinert werden, aufweist, oder in Schritt c) ein Granulat (4) direkt aus den Zwischenstücken (3) herausgearbeitet wird.

7. Verfahren nach Anspruch 6, **dadurch gekennzeichnet, dass** mehrere das Granulat (4) ausbildende Partikel (5) nach Durchführen des zweiten Teilschrittes c2) eine Korngröße zwischen 20 µm und 2000 µm aufweisen.

8. Verfahren nach einem der Ansprüche 1 bis 7, **dadurch gekennzeichnet, dass** Schritt d) ein Sintern des Granulats (4), vorzugsweise ein poröses Sintern und/oder ein selektives Lasersintern (SLS-Verfahren), umfasst.

9. Verfahren nach einem der Ansprüche 1 bis 8, **dadurch gekennzeichnet, dass** an dem Grundkörper (6) eine Oberflächenbehandlung durchgeführt wird.

## Claims

1. A method for producing an object (1) comprising plastic material and being provided for surgical use, the method comprising at least the following steps:
a) providing a plastic powder (2);
b) heating and pressing the plastic powder (1) thus forming at least one intermediate piece (3);
c) mechanically comminuting the at least one intermediate piece (3) to form a granulate (4); and
d) joining the granulate (4) to form an integral base body (6).

2. The method according to claim 1, **characterized in that** the base body (6) has a porous structure.

3. The method according to claim 2, **characterized in that** the porous structure is an open-cell or closed-cell structure.

4. The method according to any one of the claims 1 to 3, **characterized in that** the plastic powder has a grain size between 20 µm and 900 µm.

5. The method according to any one of the claims 1 to 4, **characterized in that** the at least one intermediate piece (3) is plate-shaped, has a porous material structure and/or is formed of solid material.

6. The method according to any one of the claims 1 to 5, **characterized in that** step c) includes a first partial step c1) in which the at least one intermediate piece (3) is pre-comminuted to form single pieces and a second partial step c2) in which the single pieces are further comminuted to form granulate (4), or that in step c) a granulate (4) is produced directly from the intermediate pieces (3).

7. The method according to claim 6, **characterized in that,** after carrying out the second partial step c2), plural particles (5) forming the granulate (4) have a grain size between 20 µm and 2000 µm.

8. The method according to any one of the claims 1 to 7, **characterized in that** step d) comprises sintering of the granulate (4), preferably porous sintering and/or selective laser-sintering (SLS process).

9. The method according to any one of the claims 1 to 8, **characterized in that** a surface treatment is carried out on the base body (6).

## Revendications

1. Procédé de fabrication d'un objet (1) présentant du plastique ainsi que prévu pour une utilisation chirurgicale avec au moins les étapes suivantes consistant à :
a) fournir une poudre plastique (2) ;
b) chauffer et compresser la poudre plastique (2), tout en formant au moins une pièce intermédiaire (3) ;
c) broyer mécaniquement l'au moins une pièce intermédiaire (3) en granulat (4) ; et
d) lier le granulat (4) à un corps de base (6) d'une seule pièce.

2. Procédé selon la revendication 1, **caractérisé en ce que** le corps de base (6) présente une structure poreuse.

3. Procédé selon la revendication 2, **caractérisé en ce que** la structure poreuse est une structure à alvéoles ouvertes ou fermées.

4. Procédé selon l'une quelconque des revendications 1 à 3, **caractérisé en ce que** la poudre plastique présente une granulométrie entre 20 µm et 900 µm.

5. Procédé selon l'une quelconque des revendications 1 à 4, **caractérisé en ce que** l'au moins une pièce intermédiaire (3) est réalisée en forme de plaque, présente une structure de matériau poreuse et/ou est formée à partir d'un matériau plein.

6. Procédé selon l'une quelconque des revendications 1 à 5, **caractérisé en ce que** l'étape c) présente une première partielle étape c1) dans laquelle l'au moins une pièce intermédiaire (3) est prébroyée en morceaux individuels, et une seconde étape partielle c2) dans laquelle les morceaux individuels sont en outre broyés en granulats (4), ou à l'étape c), un granulat (4) est préparé directement à partir des pièces intermédiaires (3).

7. Procédé selon la revendication 6, **caractérisé en ce que** plusieurs particules (5) réalisant le granulat (4) présentent une granulométrie entre 20 µm et 2 000 µm après exécution de la seconde étape partielle c2).

8. Procédé selon l'une quelconque des revendications 1 à 7, **caractérisé en ce que** l'étape d) comprend un frittage du granulat (4), de préférence un frittage poreux et/ou un frittage laser sélectif (procédé SLS).

9. Procédé selon l'une quelconque des revendications 1 à 8, **caractérisée en ce qu'**un traitement de surface est exécuté sur le corps de base (6).
